# EUROPEAN PATENT SPECIFICATION

(11) **EP 3 958 699 B1**
(45) Date of publication and mention of the grant of the patent: **04.03.2026**
(21) Application number: 19722160.9
(22) Date of filing: 24.04.2019
(51) Int. Cl.: A42B 3/04, A61B 5/00

(54) **A WEARABLE DEVICE AND AN ARRANGEMENT OF A WEARABLE DEVICE**
TRAGBARE VORRICHTUNG UND ANORDNUNG EINER TRAGBAREN VORRICHTUNG
DISPOSITIF PORTABLE ET AGENCEMENT D'UN DISPOSITIF PORTABLE

(43) Date of publication of application: 02.03.2022
(73) Proprietor: Northern Sports Insight and Intelligence Oy, 00290 Helsinki (FI)
(72) Inventor: LAUNES, Jyrki, 02700 Kauniainen (FI); KIVIMAA, Heidi, 02700 Kauniainen (FI)
(74) Representative: Aaltonen, Janne Lari Antero
(86) International application number: PCT/FI2019/050331
(87) International publication number: WO 2020/216986

(56) References cited:
- WO-A1-2017/198990
- US-A1- 2011 218 756
- US-A1- 2013 303 946
- US-A1- 2015 306 505
- US-A1- 2018 321 097

## Description

### Technical field

The invention relates to a wearable device and an arrangement having a wearable device.

### Background

Head injuries are potential health risks in various sports. For example, in ice hockey, which is a fast and aggressive contact sport with high velocity and intensity, head injuries are often caused by the force of contact and the head acceleration after a direct or indirect impact to the athlete's head or body. Sometimes jolt to the head can cause a concussion, a type of traumatic brain injury. Concussion may also occur from an impact to the body that causes the head to move rapidly back and forth. Concussion is considered a minor brain injury, but it may in cases have serious consequences, and should be taken seriously and treated promptly.

Based on previous head injury studies, head impact levels at which athletes' risk of injury increases are relatively well known. For example, head impact level less than 40 g very seldom cause permanent damage to an athlete, but the likelihood of permanent damage starts to rise around of 40-60 g and higher. In addition, it is known that when a head impact occurs, in most cases the resulting head movement is a combination of two types of movement, linear and rotational. However, although the risk of a head injury may be evaluated based on information obtained from an incident, it may still be difficult to immediately assess how severe a head injury is by simply looking at the injured athlete.

Increased awareness of the severity of head injuries has increased the focus on how these injuries can be better prevented, such as using better protective clothing. For example, in many sports, athletes use a head covering helmet to protect the user's head and prevent injuries. The protective effect of helmets is based on a rigid outer shell designed to distribute the impact on a wide area. They also have a soft inner lining system that softens impacts and a padding that keeps the head comfortable and sits next to the athlete's head.

Regardless of whether an athlete wears a head covering helmet, it is important to monitor the effects of head impacts and athlete's exposure to impacts. Although concussions may not cause serious health problems at the first time, the data obtained from the continuously monitoring of impacts in the head area may be useful in assessing the severity of the athlete's state of health later. In fact, studies have suggested that the consequences of repeated head impacts are cumulative, and new concussions are particularly harmful if the previous concussion has not sufficiently healed.

For the injured individuals, it is crucial to recognize the concussion immediately, to prevent injured athlete from getting further and potentially more severe injuries. Also, a correct assessment made right after an incident has proven to ensure proper management and hence diminish prolonged problems.

Prior technical solutions for detecting a force on an object are disclosed in WO 2017/198990 A1 and US 2015/0306505 A1.

### Summary of the invention

It is therefore an object of the present invention to provide a wearable device by help of which impact forces on the user's head can be detected, measured and monitored.

An object of the present invention is also to provide a wearable device by help of which acceleration of user's head can be detected, measured and monitored.

Furthermore, an object of the invention is to provide a solution for collecting data detected by a wearable device which indicates head impacts, head injuries and head impact history data in order to assess the risk levels and the severity of a potential injury to a user.

An object of the invention is also to provide a system by means of which individuals' exposure to the head impacts and head injuries can be monitored remotely.

An object of the invention is also to provide an arrangement which enables detection, measurement and monitoring of head impacts and concussions on the user's head.

A further object of the invention is to provide a solution that would enable neuropsychological testing during user monitoring.

These objects are achieved by a wearable device adapted for use with a headgear for detecting impact forces on the user's head, which comprises a flexible structure, having a top cover consisting of a plurality of layers, said layers being stacked on top of each other and laminated together to form a planar shape, wherein the top cover has a first side and a second side parallel to the first side, a bottom cover consisting of a plurality of layers, said layers being stacked on top of each other and laminated together to form a shape substantially identical to the planar shape of the top cover, wherein the bottom cover has a third side and a fourth side parallel to the third side, a battery, an electronic circuit, wherein the electronic circuit is arranged below and parallel to the second side of the top cover which is electrically connected to the battery, which electronic circuit further comprises a force sensing device configured to detect impact forces on the user's head, a processing circuit configured to receive impacts detected by said force sensing device and transmit the impacts received to a user, wherein said top cover, bottom cover, battery and electronic circuit are positioned in a stack and laminated together forming a flexible moisture proof device, wherein the battery and the electronic circuit are arranged between the top cover and bottom cover, wherein the battery is mounted on the third side of the bottom cover; the electronic circuit is embedded and secured into a recess at the interface between the top cover and the battery, and located in the center area of the wearable device so that there are no electronic parts at the peripheral regions of the covers and the peripheral regions of the battery, allowing the peripheral regions to be secured to each other; and when the top cover, the battery and the bottom cover are attached to each other, the electronic circuit is confined to a space between them, which forms a moisture proof compartment therein; and wherein the wearable device is configured to be bent to fit the desired shape so that it adapts to the shape of a surface of a padding inside the headgear, and a side of the wearable device facing the skin of the user's head, is configured to adapt along the skin of the user's head, when the wearable device worn by the user.

These objects are achieved by an arrangement for detecting impact forces on the user's head, comprising a headgear, a wearable device adapted for use with the headgear having a flexible structure, the assembly comprising a top cover consisting of a plurality of layers, said layers being stacked on top of each other and laminated together to form a planar shape, a bottom cover consisting of a plurality of layers, said layers being stacked on top of each other and laminated together to form a shape substantially identical to the planar shape of the top cover, a battery, an electronic circuit which is electrically connected to the battery, which comprises a force sensing device configured to detect impact forces on the user's head, a processing circuit configured to receive impacts detected by said force sensing device and transmit the impacts received to a user, wherein said top cover, bottom cover, battery and electronic circuit are positioned in a stack and laminated together forming a flexible moisture proof device.

In this context, the term headgear should be understood broadly, without being limited to any specific headgear type. The headgear can be, for example, any sports helmet. The headgear can be, for example, a hockey helmet, a cycling helmet, a riding helmet, a football helmet, a motorcycle helmet, a skateboard helmet or a ski / snowboard helmet. A headgear may be any head protective or covering equipment, such as helmets used in construction work, industrial work or military, or care for the elderly. The headgear may also be a headband, a sweatband, a hood, a headscarf, a top hat, a cowboy hat or a hairpin, for example.

Headgear with a wearable device according to an invention can also be used, for example, for the remote monitoring of elderly people, senior citizens, or persons who may at least partially be assisted in carrying out some of their daily tasks. The use of a wearable device for monitoring the elderly can, for example, provide some comfort for his family members and other caretakers, especially if the elderly person lives alone.

Preferred embodiments of the invention are described in the dependent claims.

### Brief description of drawings

The invention will now be described in more detail with reference to the preferred embodiments and with reference to the accompanying drawings in which:
- FIGURE 1: is a cross-sectional top view of a wearable device according to an embodiment of the present invention, wherein the wearable device is mounted inside the user's helmet;
- FIGURE 2: is a cross-sectional side view of a wearable device according to an embodiment of the present invention;
- FIGURE 3: is an exploded side view of a wearable device according to an embodiment of the present invention;

### Detailed description of the embodiments

In the following, embodiments of the invention will be described with reference to the accompanying drawings 1-3.

The following embodiments of the invention presents a wearable device adapted to be used together with a headgear and intended for detecting, measuring and monitoring impact forces on the user's head. An arrangement having a wearable device is also presented.

The wearable device comprises a flexible structure and an electronic circuit and a battery arranged therein. It comprises at least one force sensing device such as an accelerometer sensor and/or a gyroscope. Said force sensing device is designed to determine the magnitude of linear acceleration to the wearable device when worn by a user. In another embodiment said force sensing device may also be designed to determine the rotational acceleration of the impact and direction of the impact. Said force sensing device is located within the flexible structure and communicates with the surface of the user's skin and the inner surface of the headgear through the flexible structure. Since the wearable device is very thin, the distance of the force sensing device from the surface of the skin of the user's head is very small, only a few millimeters.

When a user has a headgear in his head and the headgear is equipped with a wearable device and user's head area is subjected to a hit, said force sensing device detects the impact and measures the magnitude of the impact force and records its timestamp provided that a predetermined threshold value for the impact is exceeded. The predetermined threshold value may be, for example, 30 g, 40 g or 50 g, but the value is not limited these values only and may vary. The threshold value may be predetermined as desired. When an impact is detected, the wearable device notifies the user of the impact detected by transmitting said data to a receiver unit of a user in order to display said impact data in a readable form on a receiver unit. The receiver unit may be any wireless or wired device, such as a smartphone, laptop, wristwatch, or other computer device.

When the measured data cannot be transmitted to the user's receiver unit, the data related to the impact can be stored in the internal memory of the wearable device at least until it can be retransmitted to the receiver unit. Data can also be stored in the wearable device's memory so that the measurement history of the impacts can be viewed later. The measurement data of the impact can be monitored at the desired time interval and can be used to estimate the follow-up actions of the affected user. Data stored in the internal memory may be subsequently transmitted to a remote user via a communication network, for example wirelessly, when a remote user is nearby, in the range of the wireless communication network. Thus, it enables the continuous monitoring of the individual's impact history data.

Preferably, the wearable device is a separate, battery-powered, measuring device configured to be mounted on a headgear and removable from the headgear if desired. It can be retrofitted to, for example, the inner surfaces of the headgear, preferably as near as possible the user's skin. Installation and commissioning of the wearable device can be done without removing or breaking the existing liner and/or padding and/or headgear structures. The wearable device can also be easily removed from the headgear when it is no longer in use. Installation and removal can be done without tools and without altering the original design of the headgear, which is designed to be an integral part of the headgear. When the wearable device is installed inside the headgear the wearable device also does not change the appearance of the headgear. However, although the wearable device is designed to be a separate device from a headgear, in some embodiments it may be possible to integrate the wearable device permanently into the headgear by using a suitable adhesive. In addition, although the wearable device is intended to be installed inside the headgear, it may also be possible to install the wearable device outside the headgear. For example, it may be possible to attach the wearable device to the outer shell or outer cover of the headgear, even if it is not the preferred installation location.

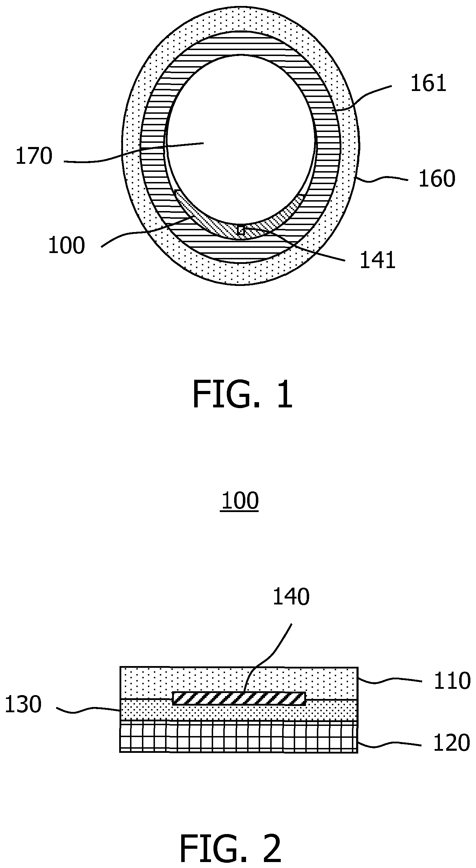

Referring to FIG. 1, there is shown a cross-sectional view of a wearable device according to an embodiment of the present invention, wherein the wearable device is mounted inside the user's helmet. The outermost part of FIG. 1 is a helmet shell 160 that covers the user's head 170. The interior of the helmet shell 160 is lined with a cushion, or padding 161, made of any suitable material such as expanded polypropylene or vinyl nitrile. The padding 161 may have a special geometry that allows it to fit comfortably into the head of the user. In FIG. 1, the wearable device 100 is arranged between the skin surface of the user's head 170 and the surface of the padding 161. The wearable device 100 may be attached to the helmet padding 161 by using a suitable adhesive such as double-sided tape. Preferably, the wearable device 100 is positioned as close as possible to the user's skin. Because the wearable device 100 is very flexible in its material, it can be bent to fit the desired shape so that it adapts to the shape of the surface of the padding 161 inside the helmet.

Correspondingly, the side of the wearable device 100 facing the skin of the user's head 170 adapts along the skin of the user's head, as shown in FIG. 1. FIG. 1 shows the mounting method in which the wearable device 100 is positioned behind the user's head. However, the shown mounting method should not be interpreted as limiting. Said wearable device may optionally be placed anywhere between the skin and the helmet padding.

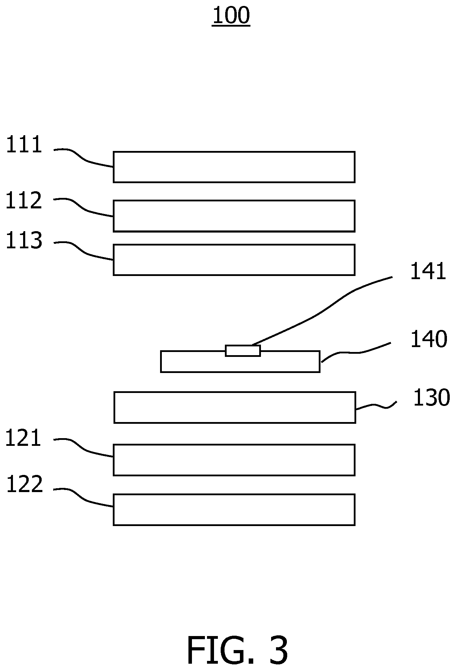

FIGS. 2 and 3 show a cross-sectional side view of a wearable device according to an embodiment of the present invention. In FIG. 2 the flexible structure of the wearable device 100 is presented. In FIG. 3, the various layers of the flexible structure of the wearable device have been depicted as physically separate from each other for clarity purposes only. Referring in FIG. 2, at the topmost part of the wearable device 100, there is a top cover 110, consisting of a plurality of layers made of a plurality of materials (not shown in FIG. 2), wherein said layers have been stacked on top of each other and laminated together to form a planar shape and having substantially uniform thickness, as shown in FIG. 2. The top cover 110 has a first side and a second side parallel to said first side, wherein the second side of the top cover 110 is mounted on top side of the electronic circuit 140. A battery 130 is located below the top cover 110 and the electronic circuit 140. At the lowermost of the structure of the wearable device 100 there is a bottom cover 120 consisting of a plurality of layers made of a plurality of materials, wherein said layers have been stacked on top of each other and laminated together to form a planar shape (not shown in FIG. 2). The bottom cover 120 may comprise different materials than the top cover 110. The top cover and the bottom cover may also be made of the same materials or at least partially of the same materials. The bottom layer 120 may have a uniform thickness throughout. The bottom cover 120 has a third side and a fourth side parallel to said third side, wherein the third side of the bottom cover is mounted on bottom side of the battery 130. Top cover 110 and battery 130 can be secured to each other by adhesive, such as glue. There is a recess at the interface between the top cover 110 and the battery 130 into which electronic circuit 140 is embedded and secured. The electronic circuit 140 is located in the center area of the wearable device 100 so that there are no electronic parts at the peripheral regions of the covers 110, 120 and the peripheral regions of the battery 130, allowing the peripheral regions 110, 120, 130 to be secured to each other. When the top cover, the battery and the bottom cover are attached to each other, the electronic circuit 140 is then confined to a space between them which forms a moisture proof compartment therein.

Referring now to FIG. 3, there is presented an exploded side view of a wearable device according to an embodiment of the present invention. According to an embodiment, the top cover 110 consists of a plurality of layers made of materials comprising, for example, at least one of the following materials: polyethylene terephthalate, polyethylene, polyurethane, vinyl nitrile, thermoplastic polyurethane, expanded polypropylene, thermoplastic polyethylene, thermoplastic rubber, leather and/or ethylene vinyl acetate and/or foam or their mixture. The choice of materials can thus have a significant effect on the properties of the top cover, for example its flexibility, breathability and suitability. Each of the layers may be of identical in their shapes, having equal width and length, for example. However, said layers need not be of the same size. For example, the edges of the top cover and bottom cover may be rounded, not rectangular in shape, when viewed from the side.

According to an embodiment the wearable device 100 comprises at least one layer of a double-sided adhesive foam tape 113, or foam, the material of the foam tape (or foam) having a thickness, for example, approximately 0.8 or 1.6 mm thick, but it is not limited to these thicknesses only. It has two sides parallel to each other. On one side there may be a permanent adhesive and on another side there may be a removable adhesive. The mounting side of the foam tape 113 facing the underlying electronic circuit 140 may thus be provided with a removable adhesive that allows the foam tape 113 to be cleanly removed from the electronic circuit 140. The side which is opposite to the mounting side, and facing away from the electronic circuit 140 may be provided with either removable or permanent adhesive for attachment. The foam tape 113 may be provided with holes. In an embodiment, said layer 113 is formed of two layers of foam tape, that are superimposed, each layer having a thickness of 0.8 mm and thus a total thickness of 1.6 mm, for example. The purpose of the foam tape 113 is to smooth out the uneven component side of the underlying electronic circuit (board), and protect the electronics from moisture, and act as a cushion. The size of a layer 113 may be for example 42 mm x 80 mm or 40 x 80 mm (width x length).

According to an embodiment of the invention, the wearable device may comprise at least two layers of foam tape, or layers of foam, stacked on top of each other wherein the foam tape 113 (or foam) closest to the electronic circuit 140 may be, for example, approximately 1.6 mm thick, and the second layer of foam tape 112 (or foam) on top of first layer may be, for example, approximately 0.8 mm thick. In an embodiment, said layer 113 may be formed of two or more layers of foam tape, that are superimposed, each layers having a thickness of 0.8 mm, for example. The thicknesses of foam tape are not however limited to the above said thicknesses only. The thickness of the foam (or foam tape) may be, for example 0.8-1.0 mm thick. Said layers of foam tape 112, 113 may cover the whole top side of said underlying electronic circuit 140 and an upper surface of the underlying battery 130 and protects them from moisture and dirt. The foam tape 112 blocks the holes in the thick foam tape 113 and protects the electronics from moisture, acts as a cushion. The size of a layer 112 may be, for example, 42 mm x 80 mm, or 40 mm x 80 mm (width x length).According to an embodiment of the invention, the surface of the top cover may be further provided with a layer of single-sided adhesive tape 111 of breathable hypoallergenic material having an adhesive surface on lower side and non-adhesive surface on top side which is parallel to its lower side. Said tape 111 may cover the surface of the underlying foam tape 112 entirely, or at least partly. It may preferably have a high skin compatibility and breathability allowing it to be placed against the skin surface of the user's head. Said tape 111 can be constructed, for example, from viscose fabric combined with a skin-friendly adhesive on a side facing towards the layer of underlying foam tape. The top cover may further be provided with a wear resistant layer disposed on one side of skin-friendly adhesive tape 111. The layer closest to user's skin may be equal in dimensions to the wear resistant layer. The thickness of a layer of single-sided adhesive tape may be between 0.1-1.0 mm, for example, 0.1 mm, 0.25 mm, 0.3 mm or 0.5 mm. The size of a layer of single-sided adhesive tape may be for example 42 mm x 80 mm or 40 x 80 mm (width x length).

The wearable device 100 further comprises an electronic circuit 140 which is arranged below and parallel to the second side of said top cover. The electronic circuit 140 has a printed circuit board (PCB) having a top side and a bottom side which is parallel with the top side. The PCB has a uniform thickness, for example 0.8 or 1.6 mm thick. It covers at least part of the top side of the underlying battery 130 and is in central region above it. The length of the PCB is preferably less than the length of the top cover and the width of the PCB is preferably less than the width of the top cover. For example, the size of the printed circuit board may be about 20 mm x 40 mm (width x length). The printed circuit board is mounted in the center region so that the peripheral region is free of the electronic circuit 140, as shown in the FIG. 2. The printed circuit board may be rectangular or circular by its shape, but the shape is not limited to any specific shape.

The electronic circuit 140 is further provided with a plurality of connections arranged at the bottom of the printed circuit board. The electronic circuit may also comprise several active or passive electronic components which are mounted and attached to the printed circuit board by solder joints. According to the embodiment of the invention, the electronic circuit 140 comprises further at least one force sensing device 141 for detecting impact forces being indicative of head impact applied to the head region of the user. The force sensing device may be an accelerometer sensor which is preferably three-axis type, but it may also be possible to use any other type of sensor, such as a gyroscope. In an embodiment, the electronic circuit may comprise both an accelerometer sensor and a gyroscope. Said sensor is mounted on the PCB of the electronic circuit 140. Optionally, the electronic circuit may comprise other types of sensors, for detecting other environmental parameters, such as, rotational direction, temperature, pressure or humidity sensors.

The electronic circuit 140 has further a processing circuit, for example, a signal processor, a microprocessor or a microcontroller, which can execute of various functions such as sensing, processing, powering and communicating, but not limited to above said functions. The processing circuit may be provided with a memory for storing instructions and data, such as data received from the force sensing device 141, e.g. accelerometer sensor or other part of the electronic circuit 140. The processing circuit is connected to the battery 130 and it is capable of selectively controlling and monitoring the power usage and lifetime of the battery 130 and delivering the electric power to other parts and the force sensing device 141. The processing circuit is electrically connected to the antenna and transmitter. The processing circuit is arranged to selectively operate according to one or more communication protocols, such as NFC, RFID, Bluetooth, Wi-Fi, WLAN, ZigBee. The processing circuit may be based, for example, on Bluetooth architecture or Bluetooth 5 System-on-Chip architecture having a processor, a transceiver, an antenna and support for Bluetooth low energy technology and 2.4 GHz proprietary protocol. The processing circuit further comprises measuring data processing means for processing the signals indicating the impact and/or direction of the impact on the head region measured by the force sensing device(s) and means for recording timestamps of the head impact signals. The processing circuit further comprises means for transmitting information to the receiver unit by using antenna and transmitter / transceiver and utilizing the preferably Bluetooth communication protocols for data transmission. Processing circuit is further configured to execute power control operations by switching the wearable device between different operation modes: sleep mode, wake-up mode and active mode of the processing circuit.

The processing circuit is preferably capable to handle any of the following: processing commands, calculation, data tracking, monitoring and reading, data storage, data analysis and data control and receiving new commands or instructions. The processing circuitry is also able to use and control the force sensing devices and collect data, i.e. the measurement results of the force sensing devices, and store them in the internal or external memory over time. The processing circuitry is also able to transmit the collected data wirelessly to the receiver unit of a remote user.

The receiver unit may be any known digital device such as a wristwatch, a heart rate monitor, a mobile phone, a laptop, a desktop computer, a wireless computing device or a portable digital assistant. Advantageously, communication between the wearable device and the receiver unit is performed wirelessly in known ways of communication, preferably by utilizing Bluetooth. Upon completion of the data transmission by the processing circuit, the receiver unit may display the information received from the wearable device, including for example, the values of the measured readings for the head impact levels or the alerts received from wearable device by a receiver unit.

Preferably, the signals to be measured by a force sensing device are processed in the processing circuit of the wearable device, for example filtered, amplified and detected, using known methods such that the signals indicative of impact force can be reliably identified from the measured signal and the signals identified can be transmitted to the receiver unit in an easily readable format. Measurement and signal processing by the processing circuit in the wearable device can be based, for example, on models where the measured signal is compared to a predetermined reference value, and if the comparison exceeds a certain threshold value, for example, a predetermined value, the measured reading is accepted as an impact on the head region.

The structure of wearable device 100 further comprises a layer having a battery 130, such as Zinc-Manganese dioxide or Lithium-ion based battery which consists of one or more electrochemical cells and is provided with connections to power the wearable device and electrical components arranged therein. The battery 130 is arranged on the bottom side of the electronic circuit 140, and is placed next to the electronic circuit. The battery is designed to be eco-friendly, thin and flexible, i.e. soft, being resistant to bending and twisting. It may be printable. The printed text or markings may be on at least one side of said battery. As an alternative to flexible battery, also non-flexible batteries may be used, such as coin cell or button cell type batteries. Said wearable device is not, however, limited to any specific type of battery, and therefore any suitable battery type may be potentially used. Said battery may be uniformly thick throughout. The battery may have a thickness which is less than 1 millimeter, for example, 0.4 mm, 0.5 mm, 0.7 mm or 0.8 mm thick. The battery thickness may also be slightly greater than 1 mm thick, for example, 1.5 mm. It has an upper side and a lower side which is parallel to its upper side. The upper side of the battery may be provided with a connecting interface, having a plurality of connectors arranged therein. In some embodiment said connection interface may be on lower side of the battery, or alternatively arranged so that the plurality of connectors of the connection interface are on either side of the battery. The lower side of the battery extends over the third side of the bottom cover and may cover the third side of the bottom cover entirely. The shape of the outer periphery of the battery may be substantially identical to the shape of the outer periphery of the bottom cover so that the edges thereof can be positioned exactly overlapping. Outer dimensions, thickness and shape of battery as well as connection terminal size and location can be tailored according to user's needs. For example, the dimensions of the battery may be the same as the dimensions of the bottom cover, for example, 40 mm x 80 mm (width x length). The weight of the battery may be in the range from 0.9 to 3.0 grams, for example 2.9 gram. The battery has, for example, 1.5-3 Volts of nominal voltage. The battery can be provided with different capacities, for example, 5 mAh, 10 mAh, 14 mAh, 18 mAh, 20-25 mAh, 30-40 mAh, 80 mAh, 90 mAh or 100mAh, the selection of the capacity being based on the user's needs. The capacity of the battery is not however limited to these capacities only, but may vary. For example, the capacity may be greater than 100 mAh. In an exemplary use, the battery may last, for example, 200-300 hours or more, after which it must be replaced or recharged if a rechargeable battery is used.

The wearable device may further comprise a thin activation tab (not shown in FIG. 3) between the battery connection interface and the connection of electronic circuit 140. The activation tab can be removed before activation of the wearable device. The purpose of the activation tab is to prevent premature closing of the electronic circuit. The wearable device may further comprise an anisotropic conductive tape, and, optionally, the double-sided adhesive tape, (not shown in FIG. 3) both of which are located between the electronic circuit and the battery, and covering one side of the electronic circuit or at least partly. The purpose of the anisotropic tape is to electrically connect and mechanically bond electronic circuits and its component to the layers of the wearable device. At the lowermost part of the wearable device 100 is a bottom cover 120.

According to an embodiment the bottom cover 120 consists of a plurality of layers of materials, comprising, for example, at least one of the following: polyethylene terephthalate, polyethylene, polyurethane, vinyl nitrile, thermoplastic polyurethane, expanded polypropylene, thermoplastic polyethylene, thermoplastic rubber, and/or ethylene vinyl acetate, and/or foam or a mixture thereof. Each of the layers may be of identical in their shapes, having equal width and length, for example. The thickness of said layers may vary. Each layer may be made of a different material, which may have a significant effect on the mechanical properties and comfort of the bottom layer.

According to an embodiment the bottom cover of the wearable device 100 comprises at least one layer of a double-sided adhesive foam tape, or foam (not shown in FIG. 3), the material of the foam having a thickness, for example, approximately 0.8-1.6 mm thick, which is arranged below the battery. The foam layer may cover one side of the battery completely or at least partially. The size of the foam tape below said battery may be, for example, 10 mm x 20 mm (width x length) or even smaller.

According to an embodiment the bottom cover comprises a layer of double-sided adhesive tape 121 of size, for example 42 mm x 80 mm (width x length), being either transparent or semitransparent and having a removable skin-friendly adhesive on one side, which layer can be manufactured of any suitable material, such a polyethylene terephthalate (PET) or polyethylene. Said layer is provided with a protective layer, for example, a thin release liner 122 made of a suitable material, such as of polyethylene (PE), and arranged to cover the second side of said layer, either entirely or at least partially to protect the underlying layers from dirt and moisture and to protect the adhesive surface until intentional attachment of the wearable device.

The layers, as described above, are positioned on the stack, attached to each other, for example, with adhesives and laminated together to form a moisture proof and flexible wearable device. Any known lamination or attachment methods may be used, such pressure and heat, or gluing. Moisture proof may refer here to moisture resistance, so that the wearable device, if it is located in a humid environment, is resistant to moisture entering it under these conditions. In another embodiment the wearable device may be waterproof, thereby making wearable device water-resistant so that it remains relatively unaffected by water or resisting the ingress of water under specified conditions.

Referring to FIGS. 2 and 3, the wearable may comprise following layers a)-h) arranged in a stack so as to overlap: a) a layer of single-sided adhesive tape 111 of breathable hypoallergenic material having an adhesive surface on lower side and non-adhesive surface on top side which is parallel to the lower side, b) optionally, a layer of double-sided adhesive foam tape 112 having a thickness, for example, of 0.8 mm, having permanent adhesive on both sides of the foam tape, c) a layer of double-sided adhesive foam tape 113 having a thickness, for example, of 1.6 mm, and having permanent adhesive on one side and removable adhesive on another side, d) an electronic circuit 140 having a printed circuit board, being either rigid or flexible printed circuit board, and a plurality of electronic component mounted therein, the electronic circuit further comprising: an accelerometer sensor and/or a gyroscope, a processing circuit connected to accelerometer sensor and/or a gyroscope, and antenna connected to processing circuit for transmitting the impact data to a user, e) a battery 130 connected to the electronic circuit for providing electrical energy to it and the electronic components, f) a layer of foam tape below one side of the battery to protect the battery, g) a layer of double-sided adhesive tape 121, being either transparent or semitransparent and having a removable skin-friendly adhesive on one side, and h) optionally, a release liner 122 covering the one side of the double-sided adhesive.

The wearable device is formed of two separate covers, both of which may be pre-fabricated to form separate multilayer arrangements, each of them preferably having equal or at least partly equal overlapping shape. It is possible that some of the layers are slightly smaller in size than the other layers, resulting in the shape of rounded corners on the edges of the covers. In this method, the top cover 110 consists of layers 111-113 and the bottom cover 120 consists of layers 121-122. Said top cover 110 can be made as a first sub-assembly, comprising, for example, above mentioned layers: a)-c), and said bottom cover 120 can be made as a second sub-assembly, comprising, for example, above mentioned layers: f)-h). The electronic circuit 140 may be the third sub-assembly and the battery 130 may be the fourth sub-assembly. Said sub-assemblies may be manufactured separately in an economical manner and combined in a final assembly of the wearable device by positioning them on the stack and laminating them together to form a moisture proof and flexible wearable device. The electronic circuit 140 (and sensor 141) is located between said layers 111-113, 130 and 121-122 and sealed so that, for example, water, moisture, particles and dirt do not enter the electronic circuit inside the device, even if the device is exposed to them.

According to an embodiment a wearable device comprises:
i) a flexible structure, comprising:
   a. a top cover consisting of layers a)-c), said layers a)-c) being stacked on top of each other and laminated together to form a planar shape;
   b. a bottom cover consisting of layers f)-h), said layers f)-h) being stacked on top of each other and laminated together to form a shape substantially identical to the planar shape of the top cover;
ii) a battery;
iii) an electronic circuit which is electrically connected to the battery, comprising:
   a. a force sensing device configured to detect impact forces on the user's head;
   b. a processing circuit configured to receive impacts detected by said force sensing device and transmit the impacts received to a user,
wherein said top cover, bottom cover, battery and electronic circuit are positioned in a stack and laminated together forming a flexible moisture proof device and where the electronic circuit is embedded and secured in a recess at the interface between the top cover and the battery.

The structure of the wearable device is described above. The following describes briefly the operation of the device when used. The wearable device 100 is designed to communicate with separate application software, for example, a mobile application, that the user can download to its receiver unit, such as a user's smartphone, when the wearable device 100 is introduced. The application software enables the sharing of information and impact history data of registered users on multiple persons. Persons with whom a registered user wants to share their information can only be specified by the registered user himself.

When the wearable device 100 is activated for use and is mounted inside the user's helmet, or headgear, impacts or collision forces on the user's helmet or head can be detected and measured by the force sensing device 141 of the wearable device 100. The wearable device 100 sends the impact data measured over time as soon as the impact on the force sensing device is sufficiently severe. For example, the wearable device 100 stores the hit if it determines that the impact force exceeds a predetermined threshold, for example 40 g or more. If an impact is detected, the impact data can also be stored in the internal memory of the wearable device 100 together with the event timestamp. The transmitter of the wearable device 100 will then start sending the measured impact data together with the event timestamp. At the same time, the transmitter sends information about the status of the battery charge level to the remote user. The wearable device 100 uses the Bluetooth protocol whereby each wireless/wired receiver unit connected to the wearable device 100 can receive the data transmitted by the wearable device. For example, if there is a receiver unit nearby, within the Bluetooth range, and application software is installed in the receiver unit, the receiver unit can then receive the information and display it to the remote user. The user can then decide on the follow-up based on the information received. The receiver unit can act as a gateway and transmits the received data to the cloud server, for example, where the data can be collected and the information provided by the force sensing device is assigned to the addressed recipients.

Although the invention has been described above with reference to the examples of the accompanying drawings, the invention is not limited thereto but can be modified in several ways within the scope of the appended claims. Therefore, all words and expressions should be interpreted broadly and are intended to illustrate, not to limit the embodiment. It will be apparent to those skilled in the art that, as technology advances, the inventive concept can be implemented in different ways. It will also be apparent to one skilled in the art that the described embodiments may be, but need not, be combined with other embodiments in different ways. For example, it will be apparent to those skilled in the art that the wearable device according to the invention described above can be used as a measuring device. It can be used in many outdoor or indoor environments to measure acceleration. The sensor may also be of a type other than an accelerometer, such as a temperature or humidity sensor. It may be possible to develop a measuring device so that it can also measure position information, for example, the location of the user can be monitored. Such a measuring device may optionally be programmed to connect to other corresponding measuring devices, whereby all these interconnected measuring devices may be arranged to collect measurement data, for example, from different age groups, sports, occupations, and transmit the collected data to a separate storage medium such as a server, cloud service or memory medium. The collected data can then be used to evaluate and analyze the groups, for which data have been collected, for example, using statistical methods.

## Claims

1. A wearable device (100) adapted for use with a headgear for detecting impact forces on a user's head (170), wherein
the wearable device (100) comprises:
i) a flexible structure comprising:
a. a top cover (110) consisting of a plurality of layers, said layers being stacked on top of each other and laminated together to form a planar shape, wherein the top cover (110) has a first side and a second side parallel to the first side;
b. a bottom cover (120) consisting of a plurality of layers, said layers being stacked on top of each other and laminated together to form a shape substantially identical to the planar shape of the top cover (110), wherein the bottom cover (120) has a third side and a fourth side parallel to the third side;
ii) a battery (130);
iii) an electronic circuit (140), wherein the electronic circuit (140) is arranged below and parallel to the second side of the top cover (110), and is electrically connected to the battery (130), the electronic circuit comprising:
a. a force sensing device (141) configured to communicate with a surface of the user's skin and an inner surface of the headgear through the flexible structure, and to detect impact forces on the user's head, and
b. a processing circuit configured to receive impact data detected from said force sensing device (140) and transmit said data to a user,
wherein the battery (130) and the electronic circuit (140) are arranged between the top cover (110) and bottom cover (120), **characterized in that** the third side of the bottom cover (120) is mounted on the bottom side of the battery (130); the electronic circuit (140) is embedded and secured into a recess at the interface between the top cover (110) and the battery (130), and located in the center area of the wearable device (100) so that there are no electronic parts at the peripheral regions of the covers (110, 120) and the peripheral regions of the battery (130), allowing the peripheral regions (110, 120, 130) to be secured to each other; and when the top cover, the battery and the bottom cover are attached to each other, the electronic circuit (140) is confined to a space between them, which forms a moisture proof compartment therein; and wherein the wearable device (100) is configured to be bent to fit the desired shape so that it adapts to the shape of a surface of a padding (161) inside the headgear, and a side of the wearable device (100) facing the skin of the user's head (170), is configured to adapt along the skin of the user's head, when the wearable device worn by the user.

2. A wearable device (100) according to claim 1, **characterized in that** at least one of the plurality layers of the top cover is of foam.

3. A wearable device (100) according to claim 1, **characterized in that** at least one of the plurality layers of the top cover (110) is of single-sided adhesive tape of breathable hypoallergenic material having an adhesive surface on lower side and non-adhesive surface on top side which is parallel to the lower side.

4. A wearable device (100) according to claim 1, **characterized in that** at least one of the plurality layers of the top cover (110) is made of leather.

5. A wearable device (100) according to claim 1, **characterized in that** at least one of the plurality layers of the bottom cover (120) is of foam.

6. A wearable device (100) according to claim 1, **characterized in that** at least one of the plurality layers of the bottom cover is of double-sided adhesive tape, being either transparent or semitransparent and having a removable skin-friendly adhesive on one side.

7. A wearable device (100) according to claim 1, **characterized in that** at least one of the plurality layers of the bottom cover (120) is a release liner.

8. A wearable device (100) according to claim 2, **characterized in that** said battery (130) is at least partly covered by at least one layer of foam.

9. A wearable device (100) according to claim 1, **characterized in that** the bottom cover is of uniform thickness.

10. A wearable device (100) according to claim 1, **characterized in that** the wearable device (100) is uniformly thick throughout.

11. A wearable device (100) according to claim 1, **characterized in that** the wearable device is a separate part of a headgear and configured to be detachable.

12. A wearable device (100) according to claim 1, **characterized in that** the processing circuit comprises a memory for storing the detected and measured head impact data and their timestamps.

13. A wearable device (100) according to claim 1, **characterized in that** the processing circuit is configured to wirelessly transmit measurement data collected from the head impacts to the receiver unit via the communication protocol.

14. An arrangement for detecting impact forces on a user's head, **characterized in that** the arrangement comprises: i) a headgear; ii) a wearable device (100) according to claim 1 adapted for use with the headgear.

## Patentansprüche

1. Tragbare Vorrichtung (100), die zur Verwendung mit einer Kopfbedeckung zum Erkennen von Aufprallkräften auf einen Kopf (170) eines Benutzers angepasst ist, wobei die tragbare Vorrichtung (100) umfasst:
i) eine flexible Struktur, umfassend:
a. eine obere Abdeckung (110), die aus einer Vielzahl von Schichten besteht, wobei die Schichten übereinander gestapelt und miteinander laminiert sind, um eine planare Form auszubilden, wobei die obere Abdeckung (110) eine erste Seite und eine zweite Seite parallel zu der ersten Seite aufweist;
b. eine untere Abdeckung (120), die aus einer Vielzahl von Schichten besteht, wobei die Schichten übereinander gestapelt und miteinander laminiert sind, um eine Form auszubilden, die im Wesentlichen identisch mit der planaren Form der oberen Abdeckung (110) ist, wobei die untere Abdeckung (120) eine dritte Seite und eine vierte Seite parallel zu der dritten Seite aufweist;
ii) eine Batterie (130);
iii) eine elektronische Schaltung (140), wobei die elektronische Schaltung (140) unterhalb und parallel zu der zweiten Seite der oberen Abdeckung (110) angeordnet ist und mit der Batterie (130) elektrisch verbunden ist, die elektronische Schaltung umfassend:
a. eine Krafterfassungsvorrichtung (141), die konfiguriert ist, um mit einer Oberfläche der Haut des Benutzers und einer Innenoberfläche der Kopfbedeckung durch die flexible Struktur zu kommunizieren und um Aufprallkräfte auf den Kopf des Benutzers zu erkennen, und
b. eine Verarbeitungsschaltung, die konfiguriert ist, um Aufpralldaten, die von der Krafterfassungsvorrichtung (140) erkannt werden, zu empfangen und die Daten an einen Benutzer zu übertragen,
wobei die Batterie (130) und die elektronische Schaltung (140) zwischen der oberen Abdeckung (110) und der unteren Abdeckung (120) angeordnet sind, **dadurch gekennzeichnet, dass** die dritte Seite der unteren Abdeckung (120) an der unteren Seite der Batterie (130) montiert ist; die elektronische Schaltung (140) in einer Vertiefung an der Grenzfläche zwischen der oberen Abdeckung (110) und der Batterie (130) eingebettet und befestigt ist und sich in dem Mittelbereich der tragbaren Vorrichtung (100) befindet, sodass keine elektronischen Teile an den peripheren Regionen der Abdeckungen (110, 120) und den peripheren Regionen der Batterie (130) vorhanden sind, wobei es den peripheren Regionen (110, 120, 130) ermöglicht wird, aneinander befestigt zu werden; und, wenn die obere Abdeckung, die Batterie und die untere Abdeckung aneinander angebracht sind, die elektronische Schaltung (140) auf einen Raum zwischen ihnen beschränkt ist, der eine feuchtigkeitsdichte Kammer darin ausbildet; und wobei die tragbare Vorrichtung (100) konfiguriert ist, um gebogen zu werden, um der gewünschten Form zu entsprechen, sodass sie sich an die Form einer Oberfläche einer Polsterung (161) innerhalb der Kopfbedeckung anpasst, und eine Seite der tragbaren Vorrichtung (100), die der Haut des Kopfs des Benutzers (170) zugewandt ist, konfiguriert ist, um sich entlang der Haut des Kopfs des Benutzers anzupassen, wenn die tragbare Vorrichtung durch den Benutzer getragen wird.

2. Tragbare Vorrichtung (100) nach Anspruch 1, **dadurch gekennzeichnet, dass** mindestens eine der Vielzahl von Schichten der oberen Abdeckung aus Schaumstoff ist.

3. Tragbare Vorrichtung (100) nach Anspruch 1, **dadurch gekennzeichnet, dass** mindestens eine der Vielzahl von Schichten der oberen Abdeckung (110) aus einseitigem Klebeband aus atmungsaktivem hypoallergenem Material ist, das eine klebende Oberfläche auf einer unteren Seite und eine nicht klebende Oberfläche auf einer oberen Seite aufweist, die parallel zu der unteren Seite ist.

4. Tragbare Vorrichtung (100) nach Anspruch 1, **dadurch gekennzeichnet, dass** mindestens eine der Vielzahl von Schichten der oberen Abdeckung (110) aus Leder hergestellt ist.

5. Tragbare Vorrichtung (100) nach Anspruch 1, **dadurch gekennzeichnet, dass** mindestens eine der Vielzahl von Schichten der unteren Abdeckung (120) aus Schaumstoff ist.

6. Tragbare Vorrichtung (100) nach Anspruch 1, **dadurch gekennzeichnet, dass** mindestens eine der Vielzahl von Schichten der unteren Abdeckung aus doppelseitigem Klebeband ist, das entweder transparent oder semitransparent ist und einen entfernbaren hautfreundlichen Klebstoff auf einer Seite aufweist.

7. Tragbare Vorrichtung (100) nach Anspruch 1, **dadurch gekennzeichnet, dass** mindestens eine der Vielzahl von Schichten der unteren Abdeckung (120) eine Trennlage ist.

8. Tragbare Vorrichtung (100) nach Anspruch 2, **dadurch gekennzeichnet, dass** die Batterie (130) mindestens teilweise durch mindestens eine Schaumstoffschicht abgedeckt ist.

9. Tragbare Vorrichtung (100) nach Anspruch 1, **dadurch gekennzeichnet, dass** die untere Abdeckung von gleichmäßiger Dicke ist.

10. Tragbare Vorrichtung (100) nach Anspruch 1, **dadurch gekennzeichnet, dass** die tragbare Vorrichtung (100) durchgehend gleichmäßig dick ist.

11. Tragbare Vorrichtung (100) nach Anspruch 1, **dadurch gekennzeichnet, dass** die tragbare Vorrichtung ein separates Teil einer Kopfbedeckung ist und konfiguriert ist, um abnehmbar zu sein.

12. Tragbare Vorrichtung (100) nach Anspruch 1, **dadurch gekennzeichnet, dass** die Verarbeitungsschaltung einen Speicher zum Speichern der erkannten und gemessenen Kopfaufpralldaten und ihrer Zeitstempel umfasst.

13. Tragbare Vorrichtung (100) nach Anspruch 1, **dadurch gekennzeichnet, dass** die Verarbeitungsschaltung konfiguriert ist, um Messdaten, die von den Kopfaufprallen erhoben werden, über das Kommunikationsprotokoll an die Empfängereinheit drahtlos zu übertragen.

14. Anordnung zum Erkennen von Aufprallkräften auf einen Kopf eines Benutzers, **dadurch gekennzeichnet, dass** die Anordnung umfasst: i) eine Kopfbedeckung; ii) eine tragbare Vorrichtung (100) nach Anspruch 1, die zur Verwendung mit der Kopfbedeckung angepasst ist.

## Revendications

1. Dispositif portable (100) adapté pour une utilisation avec un casque permettant de détecter des forces d'impact sur la tête (170) d'un utilisateur, dans lequel le dispositif portable (100) comprend :
i) une structure flexible comprenant :
a. une couverture supérieure (110) constituée d'une pluralité de couches, lesdites couches étant empilées les unes au-dessus des autres et stratifiées ensemble pour former une forme plane, dans lequel la couverture supérieure (110) a un premier côté et un deuxième côté parallèle au premier côté ;
b. une couverture inférieure (120) constituée d'une pluralité de couches, lesdites couches étant empilées les unes au-dessus des autres et stratifiées ensemble pour former une forme sensiblement identique à la forme plane de la couverture supérieure (110), dans lequel la couverture inférieure (120) a un troisième côté et un quatrième côté parallèle au troisième côté ;
ii) une batterie (130) ;
iii) un circuit électronique (140), dans lequel le circuit électronique (140) est agencé en dessous du, et parallèle au, deuxième côté de la couverture supérieure (110), et est connecté électriquement à la batterie (130), le circuit électronique comprenant :
a. un dispositif capteur de force (141) configuré pour communiquer avec une surface de la peau de l'utilisateur et une surface interne du casque à travers la structure flexible, et pour détecter des forces d'impact sur la tête de l'utilisateur, et b. un circuit de traitement configuré pour recevoir des données d'impact détectées par ledit dispositif capteur de force (140) et transmettre lesdites données à un utilisateur,
dans lequel la batterie (130) et le circuit électronique (140) sont agencés entre la couverture supérieure (110) et la couverture inférieure (120), **caractérisé en ce que** le troisième côté de la couverture inférieure (120) est monté sur le côté inférieur de la batterie (130) ; le circuit électronique (140) est intégré et fixé dans un évidement au niveau de l'interface entre la couverture supérieure (110) et la batterie (130), et localisé dans la zone centrale du dispositif portable (100) de sorte qu'il n'y a aucune pièce électronique au niveau des régions périphériques des couvertures (110, 120) et des régions périphériques de la batterie (130), permettant aux régions périphériques (110, 120, 130) d'être fixées les unes aux autres ; et lorsque la couverture supérieure, la batterie et la couverture inférieure sont attachées les unes aux autres, le circuit électronique (140) est confiné dans un espace entre eux, ce qui forme un compartiment étanche à l'humidité à l'intérieur de celui-ci ; et dans lequel le dispositif portable (100) est conçu pour être plié pour s'ajuster à la forme souhaitée de sorte qu'il s'adapte à la forme d'une surface d'un rembourrage (161) à l'intérieur du casque, et un côté du dispositif portable (100) faisant face vers la peau de la tête (170) de l'utilisateur, est conçu pour s'adapter le long de la peau de la tête de l'utilisateur, lorsque le dispositif portable est porté par l'utilisateur.

2. Dispositif portable (100) selon la revendication 1, **caractérisé en ce qu'**au moins l'une parmi la pluralité de couches de la couverture supérieure est en mousse.

3. Dispositif portable (100) selon la revendication 1, **caractérisé en ce qu'**au moins l'une parmi la pluralité de couches de la couverture supérieure (110) est en ruban adhésif simple face de matériau hypoallergénique respirant ayant une surface adhésive sur un côté inférieur et une surface non adhésive sur un côté supérieur qui est parallèle au côté inférieur.

4. Dispositif portable (100) selon la revendication 1, **caractérisé en ce qu'**au moins l'une parmi la pluralité de couches de la couverture supérieure (110) est fabriquée en cuir.

5. Dispositif portable (100) selon la revendication 1, **caractérisé en ce qu'**au moins l'une parmi la pluralité de couches de la couverture inférieure (120) est en mousse.

6. Dispositif portable (100) selon la revendication 1, **caractérisé en ce qu'**au moins l'une parmi la pluralité de couches de la couverture inférieure est en ruban adhésif double face, étant soit transparent soit semi-transparent et ayant un adhésif amovible respectueux de la peau sur un côté.

7. Dispositif portable (100) selon la revendication 1, **caractérisé en ce qu'**au moins l'une parmi la pluralité de couches de la couverture inférieure (120) est une feuille antiadhésive.

8. Dispositif portable (100) selon la revendication 2, **caractérisé en ce que** ladite batterie (130) est au moins partiellement couverte par au moins une couche de mousse.

9. Dispositif portable (100) selon la revendication 1, **caractérisé en ce que** la couverture inférieure est d'épaisseur uniforme.

10. Dispositif portable (100) selon la revendication 1, **caractérisé en ce que** le dispositif portable (100) est d'épaisseur uniforme partout.

11. Dispositif portable (100) selon la revendication 1, **caractérisé en ce que** le dispositif portable est une pièce séparée d'un casque et est conçu pour être détachable.

12. Dispositif portable (100) selon la revendication 1, **caractérisé en ce que** le circuit de traitement comprend une mémoire permettant de stocker les données d'impact de tête détectées et mesurées et leurs horodatages.

13. Dispositif portable (100) selon la revendication 1, **caractérisé en ce que** le circuit de traitement est configuré pour transmettre sans fil des données de mesure collectées par des impacts à la tête à l'unité réceptrice par l'intermédiaire du protocole de communication.

14. Agencement permettant de détecter des forces d'impact sur la tête d'un utilisateur, **caractérisé en ce que** l'agencement comprend : i) un casque ; ii) un dispositif portable (100) selon la revendication 1 adapté pour une utilisation avec le casque.
